# EUROPEAN PATENT APPLICATION

(11) **EP 3 653 718 A2**
(43) Date of publication of application: **20.05.2020**
(21) Application number: 18832640.9
(22) Date of filing: 12.07.2018
(51) Int. Cl.: C12P 3/00, C01B 25/234, C12N 9/10

(54) **METHOD FOR RECOVERING PHOSPHORIC ACID FROM FERMENTATION LIQUOR OR FERMENTATION WASTE LIQUOR, AND REUSING SAME**

(30) Priority: 13.07.2017 KR 20170089121
(71) Applicant: CJ Cheiljedang Corporation, Seoul 04560 (KR)
(72) Inventor: KIM, Jun-Woo, Seoul 04560 (KR); KIM, Jaeik, Seoul 04560 (KR); KIM, Il Chul, Seoul 04560 (KR); LEE, In Sung, Seoul 04560 (KR); KANG, Seung Hoon, Seoul 04560 (KR); KIM, Min Sup, Seoul 04560 (KR); LEE, Kang Hoon, Seoul 04560 (KR); LEE, Seung-je, Seoul 04560 (KR); LEE, Chungkwon, Seoul 04560 (KR); JUNG, Jun Young, Seoul 04560 (KR)
(74) Representative: Icosa
(86) International application number: PCT/KR2018/007915
(87) International publication number: WO 2019/013570

(57) **Abstract**

Provided is a method of recovering phosphoric acid from a fermentation broth or a waste liquid thereof and reusing the recovered phosphoric acid in fermentation.

## Description

### TECHNICAL FIELD

The present disclosure relates to a method of recovering phosphoric acid from a fermentation broth or a waste liquid thereof and a method of reusing the recovered phosphoric acid in fermentation.

### BACKGROUND ART

In some cases, phosphoric acid is essentially required to produce a target substance by fermentation. For example, International Patent Application Publication No. WO 2014/182125 discloses that a phosphoric acid source may be provided for fermentation of *O*-phosphoserine which is a precursor used to produce L-cysteine. Further, International Patent Application Publication No. WO 2014/064244 discloses a method of producing methionine from *O*-phosphohomoserine by using a sulfide and a converting enzyme. Based thereon, it can be confirmed that a phosphate is essential as a phosphorus source to produce *O*-phosphohomoserine, which is a precursor of methionine, by fermentation. Therefore, there is a need to develop a method of recovering residual phosphoric acid from a fermentation broth or a waste liquid thereof comprising a large amount of a phosphate and reusing the recovered phosphoric acid as a phosphorus source.

Conventional processes of recovering phosphoric acid are generally performed by isolating phosphoric acid from phosphoric acid-containing rock materials using an organic solvent extraction process, and recovering phosphoric acid through a neutralization process with a calcium salt, an ammonium salt, a potassium salt, a sodium salt, or the like (US3375068, US3466141A, US4543239, EP0087323A1, US7687046, and US8658117). However, the organic solvent extraction process is economically inefficient since processing costs increase by using the solvent and is complicated since a procedure of recovering the solvent needs to be additionally performed. Further, an available organic solvent may act as a toxin in a fermentation process even in a small amount, and thus it is difficult to apply the organic solvent to the fermentation process. Meanwhile, a process of recovering or producing phosphoric acid from a waste liquid of a fermentation process rather than from phosphoric acid-containing rock materials by a method without using an organic solvent has not yet been reported in patent documents or references.

### DESCRIPTION OF EMBODIMENTS

### TECHNICAL PROBLEM

The present inventors have made intensive efforts to develop a method of recovering phosphoric acid from a fermentation broth or fermentation waste liquid, and as a result, have found that a phosphate can be recovered from a fermentation broth or fermentation waste liquid without using an organic solvent, and by reusing the recovered phosphate in fermentation, a fermentation product can be obtained in an amount similar to that obtained using pure phosphoric acid, thereby completing the present disclosure.

### TECHNICAL SOLUTION

An object of the present disclosure is to provide a method of recovering phosphoric acid from a fermentation broth or fermentation waste liquid and reusing the same.

Another object of the present disclosure is to provide a method of recovering phosphoric acid from an *O*-phosphoserine (OPS) fermentation broth; or a fermentation waste liquid in which cysteine or a derivative thereof is removed and reusing the same.

Another object of the present disclosure is to provide a method of recovering phosphoric acid from an *O*-phosphohomoserine (OPHS) fermentation broth; or a fermentation waste liquid in which methionine or a derivative thereof is removed and reusing the same.

Another object of the present disclosure is to provide a method of recovering phosphoric acid from a fermentation waste liquid containing phosphoric acid.

### ADVANTAGEOUS EFFECTS OF INVENTION

In the case where phosphoric acid is recovered from a fermentation process, not from rock materials, a method of recovering phosphoric acid by a method other than an organic solvent extraction method, and reusing the recovered phosphoric acid in a fermentation process has been identified for the first time in the present disclosure. According to the method of recovering phosphoric acid from a fermentation medium and reusing the same in the present disclosure, phosphoric acid can be recovered from a fermentation broth or a fermentation waste liquid without using an organic solvent and reused in a fermentation process. Therefore, problems caused by using an organic solvent, such as acting as a toxin in a fermentation process, additional costs incurred for using the solvent, and complicating a process due to addition of a solvent-recovering process, can be overcome, and thus, the method according to the present disclosure can be effectively used in a fermentation process.

### BRIEF DESCRIPTION OF DRAWINGS

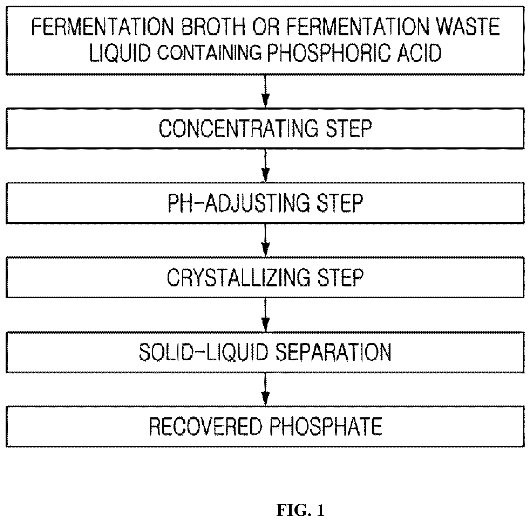

FIG. 1 briefly schematizes a process of recovering a phosphate from a fermentation broth or fermentation waste liquid containing phosphoric acid.

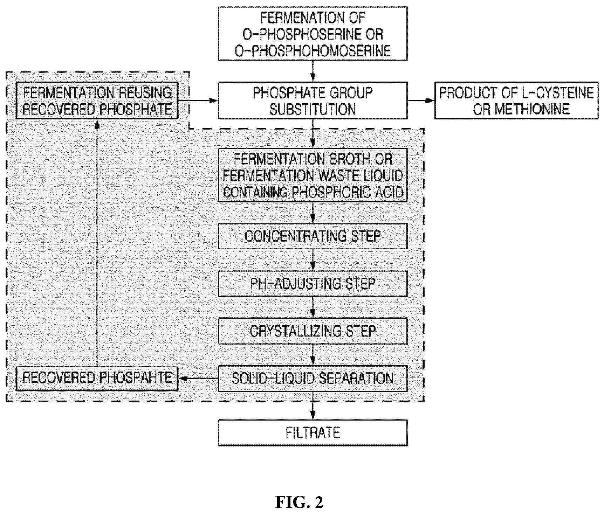

FIG. 2 briefly schematizes a process of recovering phosphoric acid from a fermentation broth which was obtained by using an enzymatic conversion reaction in an *O*-phosphoserine (OPS) or *O*-phosphohomoserine (OPHS) fermentation broth; or from a fermentation waste liquid which was obtained by removing cysteine, a cysteine derivative, methionine, or a methionine derivative from the fermentation broth; and reusing the recovered phosphoric acid in the fermentation of O-phosphoserine or O-phosphohomoserine.

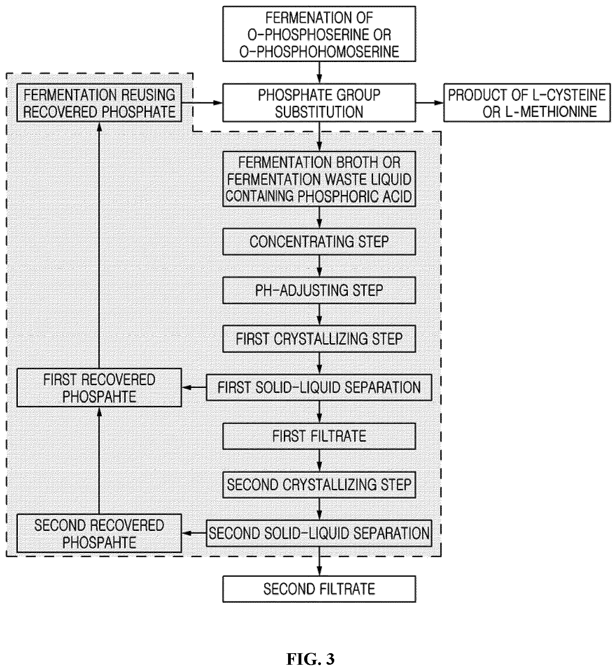

FIG. 3 briefly schematizes a process of recovering phosphoric acid via a two-step process from a fermentation broth which was obtained by using an enzymatic conversion reaction in an *O*-phosphoserine (OPS) or *O*-phosphohomoserine (OPHS) fermentation broth; or from a fermentation waste liquid which was obtained by removing cysteine, a cysteine derivative, methionine, or a methionine derivative from the fermentation broth; and reusing the recovered phosphoric acid in the fermentation of O-phosphoserine or O-phosphohomoserine.

### BEST MODE

To achieve the objects of the present disclosure, an aspect of the present disclosure provides a method of recovering phosphoric acid from a fermentation broth or a fermentation waste liquid and reusing the recovered phosphoric acid, the method comprising:
(a) concentrating a fermentation broth or a fermentation waste liquid containing phosphoric acid (concentrating step);
(b) adjusting the pH of the concentrate in the range of 8 to 11 (pH-adjusting step);
(c) crystallizing a phosphate in the concentrate having the adjusted pH and separating the crystalized phosphate from a mother liquor (crystallizing step); and
(d) using the crystallized phosphate in a fermentation medium as a phosphoric acid source (reusing step).

In the present disclosure, the fermentation broth of the step (a) (concentrating step) may refer to a medium obtained by culturing a microorganism that produces a fermentation product in a phosphoric acid-containing medium; a culture comprising the microorganism cultured with the medium; or an enzymatic conversion solution thereof.

The type of the 'fermentation product' is not limited so long as the fermentation broth comprises phosphoric acid, but specifically, may be a target substance, a derivative or precursor thereof, which are produced by fermentation. Also, the target substance may be an amino acid such as cysteine or methionine, without being limited thereto. Also, the derivative of the target substance may be a derivative of an amino acid, specifically, a derivative of cysteine or methionine. More specifically, the derivative of cysteine may comprise at least one selected from the group consisting of *N*-acetyl-cysteine, cystine, a metal-cysteine complex (*e.g*., zinc-cysteine complex, manganese-cysteine complex, iron-cysteine complex, copper-cysteine complex, and magnesium-cysteine complex) and a cysteine salt (*e.g*.: cysteine hydrochloride), without being limited thereto. The derivative of methionine may comprise at least one selected from the group consisting of *N*-acetyl-methionine, a metal-methionine complex (*e.g*., zinc-methionine complex, manganese-methionine complex, iron-methionine complex, copper-methionine complex, and magnesium-methionine complex), and a methionine salt, without being limited thereto. Also, the precursor of the target substance may be a precursor of an amino acid, specifically, a precursor of cysteine or methionine. More specifically, the precursor may be O-phosphoserine (OPS) or O-phosphohomoserine (OPHS), without being limited thereto.

In addition, the 'fermentation broth' may comprise (a) a fermentation broth of a target substance or a precursor or derivative thereof comprising phosphoric acid, (b) a fermentation broth comprising a target substance or derivatives thereof, produced by a strain producing a precursor of the target substance, a substrate, and a converting enzyme; and phosphoric acid, or (c) a fermentation broth comprising a target substance or derivatives thereof, produced by adding a converting enzyme or a microorganism expressing the converting enzyme; and a substrate to a fermentation broth of a precursor of the target substance; and phosphoric acid, without being limited thereto.

More specifically, the fermentation broth may be (i) an *O*-phosphoserine fermentation broth containing phosphoric acid, (ii) an *O*-phosphohomoserine fermentation broth containing phosphoric acid, or (iii) a fermentation broth comprising an amino acid or derivatives thereof, prepared by adding a converting enzyme or a microorganism expressing the same; and a sulfide to the fermentation broth; and phosphoric acid. In addition, the fermentation broth (iii) may be specifically a fermentation broth comprising cysteine or derivatives thereof, prepared by adding O-phosphoserine sulfhydrylase (OPSS) or a microorganism expressing the same; and a sulfide to the *O*-phosphoserine fermentation broth; and phosphoric acid. Also, the fermentation broth (iii) may be a fermentation broth comprising methionine or derivatives thereof, prepared by adding a O-Phospho-homoserine dependent (OPHS-dependent) methionine synthase or a microorganism expressing the same; and a sulfide to the *O*-phosphohomoserine fermentation broth; and phosphoric acid.

As used herein, the term 'fermentation waste liquid' may be a solution obtained by partially or entirely separating and removing the fermentation product from the fermentation broth, without being limited thereto. Specifically, the fermentation waste liquid may be a solution obtained by partially or entirely separating and removing an amino acid or derivatives or precursors thereof from a fermentation broth comprising the amino acid or derivatives or precursors thereof and phosphoric acid, without being limited thereto. For example, the fermentation waste liquid may be a solution obtained by partially or entirely separating and removing an amino acid or derivatives thereof from a fermentation broth comprising the amino acid and derivatives thereof and phosphoric acid or a solution obtained by partially or entirely separating and removing an amino acid or derivatives thereof from a fermentation broth comprising the amino acid or derivatives thereof, produced by adding a substrate and a converting enzyme, or a microorganism producing the converting enzyme to a fermentation broth of a precursor of the amino acid, and phosphoric acid. More specifically, the fermentation waste liquid may be a solution obtained by partially or entirely separating and removing cysteine or derivatives thereof, prepared by adding O-phosphoserine sulfhydrylase (OPSS) or a microorganism expressing the same; and a sulfide to the O-phosphoserine fermentation broth, from a fermentation broth comprising cysteine or derivatives thereof; and phosphoric acid, or a solution obtained by partially or entirely separating and removing methionine or derivatives thereof, prepared by adding OPHS-dependent methionine synthase or a microorganism expressing the same; and a sulfide to the O-phosphohomoserine fermentation broth, from a fermentation broth comprising methionine or derivatives thereof, and phosphoric acid, without being limited thereto.

As used herein, the term 'concentrating' of the concentrating step may be performed by any known method without limitation so long as the concentration of phosphate ions contained in the fermentation broth or fermentation waste liquid is increased by the method. Specifically, the concentrating may be performed by evaporating, heating, depressurizing, ventilating, freezing, or the like, without being limited thereto.

In the present disclosure, the concentrating is performed to increase a recovery rate of phosphoric acid, and the fermentation broth or fermentation waste liquid may be concentrated in the concentrating step such that the concentration of phosphate ions contained in the concentrate having the adjusted pH may be 60 g/L or more or 70 g/L or more, and more particularly, in the range of 60 g/L to 300 g/L or 70 g/L to 250 g/L, without being limited thereto.

In addition, in the present disclosure, before the concentrating step, the pH of the fermentation broth or fermentation waste liquid may be adjusted. Specifically, the pH of the fermentation broth or fermentation waste liquid may be adjusted to 7 or more, 7.5 or more, 8 or more, 8.5 or more, or 9 or more, more particularly, in the range of 8 to 11. By adjusting the pH before the concentrating step, an amount of ammonium ion impurities contained in the recovered phosphate may be reduced.

In the present disclosure, the step (b) (pH-adjusting step) is a step of adjusting the pH of the concentrate before crystallizing the phosphate. The pH of the concentrate is not limited so long as the phosphate is crystallized at the pH, but may be adjusted between a neutral level and an alkaline level, specifically, to 7 or more, 7.5 or more, 8 or more, 8.5 or more, or 9 or more, more specifically, in the range of 8 to 11.

In addition, in the present disclosure, the pH-adjusting step may be performed by adding an alkaline substance, specifically a hydroxide, to the concentrate. Specifically, the hydroxide may be sodium hydroxide or an aqueous solution of sodium hydroxide, without being limited thereto.

In the present disclosure, the step (c) (crystallizing step) is a step of recovering the phosphate from the concentrate having the adjusted pH, which is obtained in the pH-adjusting step. In the present disclosure, the recovery of the phosphate is performed by crystallizing the phosphate in the concentrate having the adjusted pH and separating the generated crystals from a mother liquor without using an organic solvent.

For crystallization of the phosphate, temperature may be adjusted and/or crystal nucleus may be added thereto, without being limited thereto. Specifically, for crystallization of the phosphate, a step of cooling the concentrate having the adjusted pH may further be conducted between the pH-adjusting step and the crystallizing step; or in the crystallizing step. More specifically, the concentrate having the adjusted pH may be left to stand at room temperature, the concentrate having the adjusted pH may be cooled before the crystallizing step, or the concentrate having the adjusted pH may be cooled in the crystallizing step, without being limited thereto. Meanwhile, although the recovery rate of phosphoric acid may be affected by cooling temperature, the cooling temperature is not limited so long as crystals of the phosphate are formed. Specifically, the cooling temperature may be 50°C or less, specifically in the range of 0°C to 30°C, more specifically in the range of 10°C to 20°C, without being limited thereto.

In addition, specifically, for crystallization of the phosphate, a step of adding the crystals of the phosphate as crystal nuclei may further be conducted between the pH-adjusting step and the crystallizing step or in the crystallizing step, without being limited thereto.

As used herein, the term 'phosphate' may refer to a salt of phosphoric acid or a hydrate thereof, but the type of the salt is not limited thereto so long as phosphoric acid may be recovered by crystallization. Specifically, the phosphate may comprise at least one selected from trisodium phosphate, disodium hydrogen phosphate, sodium dihydrogen phosphate, and hydrates thereof, more specifically, disodium hydrogen phosphate or hydrates thereof, without being limited thereto. The number of water molecules bound to the hydrate is not limited so long as the hydrate of the phosphate is crystallized, but may be, specifically, 1, 2, 3, 4, 5, 6, 7, 8, 9, 10, 11, 12, or 13 or more. In addition, the hydrate may be specifically, disodium hydrogen phosphate heptahydrate or disodium hydrogen phosphate dodecahydrate, without being limited thereto.

In the present disclosure, the terms phosphoric acid and phosphate may be used interchangeably.

In the present disclosure, any method enabling solid-liquid separation may be used to separate the generated phosphate crystals without limitation, specifically, a centrifuge, a filter press, a compression filter, a rotary vacuum filter, a membrane separator, or the like may be used, without being limited thereto.

In the present disclosure, the mother liquor refers to a solution obtained by separating and removing the generated phosphate crystals from the concentrate having the adjusted pH and may comprise uncrystallized phosphate ions, without being limited thereto.

In order to increase the recovery rate of phosphoric acid, the method according to the present disclosure may further comprise a second crystallizing step of re-crystallizing the phosphate from the mother liquor. According to the present disclosure, a high recovery rate of phosphoric acid may be obtained by adding phosphate crystals obtained in the second crystallizing step (second recovered phosphate) to phosphate crystals obtained in the previous crystallizing step (first recovered phosphate). Specifically, the second crystallizing step may comprise (i) concentrating a mother liquor (re-concentrating step), (ii) adjusting the pH of the mother liquor (pH re-adjusting step), and (iii) crystallizing the phosphate in the mother liquor having the adjusted pH and separating the crystalized phosphate therefrom (re-crystallizing step). The steps (i) to (iii) may be performed in the same manner as the concentrating step, the pH-adjusting step, and the crystallizing step described above. Specifically, the pH of the mother liquor may be adjusted to 7 or more, 7.5 or more, 8 or more, 8.5 or more, or 9 or more, more specifically, in the range of 8 to 11.

In addition, specifically, the second crystallizing step may further comprise adding phosphate crystals, as crystal nuclei, to the mother liquor, for example, between the pH re-adjusting step and the re-crystallizing step or in the re-crystallizing step, without being limited thereto.

In addition, according to the present disclosure, the crystallizing step may be performed more than twice to increase the recovery rate of phosphoric acid.

The method according to the present disclosure may further comprise adding an alcohol in the pH-adjusting step; or between the pH-adjusting step and the crystallizing step. Due to the low solubility of a phosphate in alcohol, the alcohol, when mixed with a phosphate solution, reduces the solubility of the phosphate in the solution, thereby increasing the recovery rate of phosphoric acid. The alcohol is not limited so long as the recovery rate of phosphoric acid is increased thereby, but may particularly be methanol, ethanol, propanol, butanol, or any combination thereof.

In the present disclosure, the step (d) (reusing step) is a step of using the phosphate crystals recovered from the crystallizing step as a source of phosphoric acid and/or phosphorus in various fermentation media requiring a source of phosphoric acid and/or phosphorus. The fermentation medium according to the present disclosure is not limited so long as the medium is used in fermentation requiring a source of phosphoric acid and/or phosphorus, but may particularly be, a fermentation medium used in production of *O*-phosphoserine or *O*-phosphohomoserine.

Another aspect of the present disclosure provides a method of recovering phosphoric acid from a fermentation broth or a fermentation waste liquid and reusing the recovered phosphoric acid, the method comprising:
(a) producing *O*-phosphoserine (OPS) using a microorganism in a fermentation medium containing phosphoric acid (OPS fermentation step);
(b) preparing a fermentation broth of cysteine or a derivative thereof by reacting O-phosphoserine produced in the step (a) with a sulfide in the presence of O-phosphoserine sulfhydrylase (OPSS) or a microorganism expressing the OPSS (converting step);
(c) concentrating the fermentation broth; or a fermentation waste liquid obtained by removing cysteine or derivatives thereof from the fermentation broth (concentrating step);
(d) adjusting the pH of the concentrate in the range of 8 to 11 (pH-adjusting step);
(e) crystallizing a phosphate from the concentrate having the adjusted pH and separating the crystalized phosphate from a mother liquor (crystallizing step); and
(f) using the crystallized phosphate in a fermentation medium as a phosphoric acid source (reusing step).

In the present disclosure, the step (a) (OPS fermentation step) is a step of producing *O*-phosphoserine by using a fermentation medium containing phosphoric acid and a microorganism. *O*-phosphoserine is an ester of serine and phosphoric acid, and phosphoric acid is required to produce *O*-phosphoserine. Also, the microorganism may be any known microorganism capable of producing *O*-phosphoserine, and examples of the microorganism may be, but are not limited to, microorganisms having enhanced activity of exporting *O*-phosphoserine (International Patent Application Publication Nos. WO 2014/182125 and WO 2014/182119). In addition, examples of microorganisms having high *O*-phosphoserine-producing ability, may be a microorganism having reduced activity of endogenous phosphoserine phosphatase (SerB) and/or enhanced activity of phosphoglycerate dehydrogenase (SerA) and/or phosphoserine aminotransferase (SerC) (International Patent Application Publication No. WO 2012/053794), without being limited thereto.

In the present disclosure, the step (b) (converting step) is a step of converting *O*-phosphoserine, obtained in the OPS fermentation step, into cysteine or derivatives thereof by reacting *O*-phosphoserine with a sulfide under the catalytic action of the *O*-phosphoserine sulfhydrylase.

In the present disclosure, the conversion reaction may also be performed by using a microorganism expressing *O*-phosphoserine sulfhydrylase, as well as the enzyme. The enzyme and the microorganism expressing the enzyme may be obtained by any means and method known in the art. Specifically, the enzyme, *O*-phosphoserine sulfhydrylase may be any known enzyme disclosed in International Patent Application Publication Nos. WO 2013/089478, WO 2012/053794, and WO 2012/053777, without being limited thereto.

Also, in the present disclosure, the sulfide is not limited so long as the sulfide reacts with *O*-phosphoserine under the catalytic action of *O*-phosphoserine sulfhydrylase, but may be at least one selected from the group consisting of CH₃SH, Na₂S, NaSH, (NH₄)₂S, H₂S, and Na₂S₂O₃.

In the present disclosure, the steps (c) to (e) are the same as the concentrating step, the pH-adjusting step, and the crystallizing step described above.

In the present disclosure, the step (f) (reusing step) is a step of using the phosphate crystals recovered from the crystallizing step in a fermentation medium requiring a source of phosphoric acid and/or phosphorus as the source of phosphoric acid and/or phosphorus. Specifically, the fermentation medium may be any fermentation medium used to produce *O*-phosphoserine, without being limited thereto.

Another aspect of the present disclosure provides a method of recovering phosphoric acid from a fermentation broth or a fermentation waste liquid and reusing the recovered phosphoric acid, the method comprises:
(a) producing *O*-phosphohomoserine (OPHS) using a microorganism in a fermentation medium containing phosphoric acid (OPHS fermentation step);
(b) preparing a fermentation broth of methionine or a derivative thereof by reacting *O*-phosphohomoserine produced in the step (a) with a sulfide in the presence of *O*-phosphohomoserine-dependent methionine synthase or a microorganism expressing the *O*-phosphohomoserine-dependent methionine synthase (converting step);
(c) concentrating the fermentation broth; or a fermentation waste liquid obtained by removing methionine or a derivative thereof from the fermentation broth (concentrating step);
(d) adjusting the pH of the concentrate in the range of 8 to 11 (pH-adjusting step);
(e) crystallizing the phosphate in the concentrate having the adjusted pH and separating the crystalized phosphate from a mother liquor (crystallizing step); and
(f) using the crystallized phosphate as a phosphoric acid source in a fermentation medium (reusing step).

In the present disclosure, the step (a) (OPHS fermentation step) is a step of producing *O*-phosphohomoserine by using a fermentation medium containing phosphoric acid and a microorganism. *O*-phosphohomoserine is an ester of threonine and phosphoric acid, and phosphoric acid is required to produce *O*-phosphohomoserine. Also, the microorganism may be any known microorganism capable of producing *O*-phosphohomoserine.

In the present disclosure, the step (b) (converting step) is a step of converting *O*-phosphohomoserine into methionine or derivatives thereof by reacting *O*-phosphohomoserine obtained from the OPHS fermentation step with a sulfide under the catalytic action of the OPHS-dependent methionine synthase.

In the present disclosure, the conversion reaction may also be performed by using a microorganism expressing OPHS-dependent methionine synthase, as well as the enzyme. Specifically, the enzyme and the microorganism expressing the enzyme may be obtained by any means and method known in the art, for example, by a method disclosed in International Patent Application Publication No. WO 2014/064244, without being limited thereto.

Also, in the present disclosure, the sulfide is not limited so long as the sulfide reacts with *O*-phosphohomoserine under the catalytic action of OPHS-dependent methionine synthase, but may comprise at least one selected from CH₃SH, Na₂S, NaSH, (NH₄)₂S, H₂S, and Na₂S₂O₃.

In the present disclosure, the steps (c) to (e) are the same as the concentrating step, the pH-adjusting step, and the crystallizing step described above.

In the present disclosure, the step (f) (reusing step) is a step of using the phosphate crystals recovered from the crystallizing step in a fermentation medium requiring a source of phosphoric acid and/or phosphorus as the source of phosphoric acid and/or phosphorus. Specifically, the fermentation medium may be any fermentation medium used to produce O-phosphohomoserine, without being limited thereto.

Another aspect of the present disclosure provides a method of recovering phosphoric acid from a fermentation waste liquid.

Specifically, the method according to the present disclosure comprises:
(a) concentrating a fermentation waste liquid containing phosphoric acid (concentrating step);
(b) adjusting the pH of the concentrate in the range of 8 to 11 (pH-adjusting step);
(c) crystallizing the phosphate from the concentrate having the adjusted pH (crystallizing step); and
(d) separating the crystallized phosphate from a mother liquor (separating step).

In the present disclosure, the fermentation waste liquid is as described above and may comprise a waste liquid of enzymatic conversion.

In the present disclosure, the steps (a) to (c) are the same as the concentrating step, the pH-adjusting step, and the crystallizing step described above.

In the present disclosure, a method of separating phosphate crystals from the mother liquor of the step (d) (separating step) is as described above in the crystallizing step.

### MODE OF DISCLOSURE

Hereinafter, the present disclosure will be described in more detail with reference to the following examples. However, these examples are for illustrative purposes only and are not intended to limit the scope of the present disclosure.

### Example 1. Recovery of Phosphoric Acid According to Concentration of O-phosphoserine fermentation waste liquid

### Example 1-1. Recovery of Phosphoric Acid Using O-phosphoserine fermentation Waste Liquid

After an *O*-phosphoserine fermentation broth was obtained by culturing a microorganism capable of producing *O*-phosphoserine (OPS) in a fermentation medium comprising phosphoric acid, the fermentation broth was reacted with a sulfide using *O*-phosphoserine sulfhydrylase (OPSS) to obtain a fermentation broth comprising cysteine or cystine (International Patent Application Publication No. WO 2012/053794). Cysteine or cystine was crystallized in the fermentation broth and separated therefrom by solid-liquid separation to obtain a fermentation waste liquid.

The *O*-phosphoserine fermentation waste liquid had an ion composition (g/L) of 24.4 of sodium ions, 6.4 of ammonium ions, 14.8 of chlorine ions, 3.4 of sulfate ions, and 34.3 of phosphate ions. 1665 ml of water was evaporated from 2000 ml of an initial waste liquid, and 50% (w/w) aqueous solution of sodium hydroxide (38.0 ml), was added thereto until the pH reached 9.00. This solution had a phosphate ion concentration of 185.4 g/L. The solution was cooled down to 15°C to obtain disodium hydrogen phosphate heptahydrate. The slurry was filtered using a centrifugal basket filter and washed with pure water.

A weight of a final product was 159.5 g. The final product had an ion composition (g/kg) of 124.6 of sodium ions, 5.3 of ammonium ions, 5.4 of chlorine ions, 4.2 of sulfate ions, and 388.5 of phosphate ions. A moisture content of the final product was 45.7 wt%. A total recovery rate of phosphoric acid was 79.2 wt%.

### Example 1-2. Recovery of Phosphoric Acid Using O-phosphoserine Fermentation Waste Liquid

A waste liquid based on the process of *O*-phosphoserine fermentation according to the present example had an ion composition (g/L) of 24.7 of sodium ions, 6.3 of ammonium ions, 16.4 of chlorine ions, 3.9 of sulfate ions, and 35.8 of phosphate ions. 1555 ml of water was evaporated from 1945 ml of an initial waste liquid, and 50% (w/w) aqueous solution of sodium hydroxide (46.5 ml), was added thereto until the pH reached 9.00. This solution had a phosphate ion concentration of 159.7 g/L. The solution was cooled down to 15°C to obtain disodium hydrogen phosphate heptahydrate. The slurry was filtered using a centrifugal basket filter and washed with pure water.

A weight of a final product was 132.3 g. The final product had an ion composition (g/kg) of 127.4 of sodium ions, 10.9 of ammonium ions, 5.2 of chlorine ions, 3.1 of sulfate ions, and 358.6 of phosphate ions. A moisture content of the final product was 49.6 wt%. A total recovery rate of phosphoric acid was 68.0 wt%.

### Example 1-3. Recovery of Phosphoric Acid Using O-phosphoserine Fermentation Waste Liquid

A waste liquid based on the process of *O*-phosphoserine fermentation according to the present example had an ion composition (g/L) of 24.6 of sodium ions, 6.2 of ammonium ions, 16.3 of chlorine ions, 3.5 of sulfate ions, and 35.6 of phosphate ions. 1315 ml of water was evaporated from 1975 ml of an initial waste liquid, and 50% (w/w) aqueous solution of sodium hydroxide (43.0 ml), was added thereto until the pH reached 9.01. This solution had a phosphate ion concentration of 99.9 g/L. The solution was cooled down to 15°C to obtain disodium hydrogen phosphate dodecahydrate. The slurry was filtered using a centrifugal basket filter and washed with pure water.

A weight of a final product was 186.0 g. The final product had an ion composition (g/kg) of 110.7 of sodium ions, 13.5 of ammonium ions, 2.4 of chlorine ions, 2.8 of sulfate ions, and 264.7 of phosphate ions. A moisture content of the final product was 60.8 wt%. A total recovery rate of phosphoric acid was 70.1 wt%.

### Example 1-4. Recovery of Phosphoric Acid Using O-phosphoserine Fermentation Waste Liquid

A waste liquid based on the process of *O*-phosphoserine fermentation according to the present example had an ion composition (g/L) of 24.5 of sodium ions, 6.2 of ammonium ions, 16.3 of chlorine ions, 3.7 of sulfate ions, and 35.6 of phosphate ions. 980 ml of water was evaporated from 1955 ml of an initial waste liquid, and 50% (w/w) aqueous solution of sodium hydroxide (42.0 ml), was added thereto until the pH reached 9.04. This solution had a phosphate ion concentration of 68.4 g/L. The solution was cooled down to 15°C to obtain disodium hydrogen phosphate dodecahydrate. The slurry was filtered using a centrifugal basket filter and washed with pure water.

A weight of a final product was 172.9 g. The final product had an ion composition (g/kg) of 112.8 of sodium ions, 20.2 of ammonium ions, 1.2 of chlorine ions, 2.6 of sulfate ions, and 255.9 of phosphate ions. A moisture content of the final product was 61.9 wt%. A total recovery rate of phosphoric acid was 63.6 wt%.

Therefore, it was confirmed that as the concentration of phosphate ions contained in the concentrate having the adjusted pH decreases, the recovery rate of phosphoric acid decreases.

### Example 2. Recovery of Phosphoric Acid According to Cooling Temperature

A waste liquid based on the process of O-phosphoserine fermentation according to the present example had an ion composition (g/L) of 23.8 of sodium ions, 6.5 of ammonium ions, 15.1 of chlorine ions, 3.4 of sulfate ions, and 35.0 of phosphate ions. 1600 ml of water was evaporated from 2000 ml of an initial waste liquid (at a temperature of 50°C or higher), and 50% (w/w) aqueous solution of sodium hydroxide (39.5 ml), was added thereto until the pH reached 9.00. This solution had a phosphate ion concentration of 159.4 g/L. The experimental condition was similar to that of Example 1-2. The solution was cooled down to 25°C to obtain disodium hydrogen phosphate heptahydrate. The slurry was filtered using a centrifugal basket filter and washed with pure water.

A weight of a final product was 110.6 g. The final product had an ion composition (g/kg) of 126.8 of sodium ions, 13.8 of ammonium ions, 2.7 of chlorine ions, 2.2 of sulfate ions, 393.9 of phosphate ions. A moisture content of the final product was 43.9 wt%. A total recovery rate of phosphoric acid was 62.2 wt%. This result indicates that a lower temperature is preferred to obtain a higher recovery rate of phosphoric acid.

### Example 3. Recovery of Phosphoric Acid by Additionally Adjusting pH before Concentrating

A waste liquid based on the process of *O*-phosphoserine fermentation according to the present example had an ion composition (g/L) of 23.4 of sodium ions, 6.4 of ammonium ions, 14.9 of chlorine ions, 3.5 of sulfate ions, and 34.7 of phosphate ions. Before an evaporation process, 50% (w/w) aqueous solution of sodium hydroxide (39.5 ml), was added thereto until the pH reached 9.02. Then, 1600 ml of water was evaporated from 2000 ml of an initial waste liquid. The pH of the solution after evaporation was 7.06 since ammonia was evaporated. Thus, 50% (w/w) aqueous solution of sodium hydroxide (16.0 ml), was further added thereto until the pH reached 9.00. The phosphate ion concentration of the solution was 152.4 g/L. The concentration condition was similar to that of Example 1-2. The solution was cooled down to 15°C to obtain disodium hydrogen phosphate heptahydrate. The slurry was filtered using a centrifugal basket filter and washed with pure water.

A weight of a final product was 141.6 g. The final product had an ion composition (g/kg) of 156.8 of sodium ions, 0.6 of ammonium ions, 1.1 of chlorine ions, 3.2 of sulfate ions, and 338.8 of phosphate ions. A moisture content of the final product was 51.2 wt%. A total recovery rate of phosphoric acid was 69.1 wt%. According to this method, the amount of ammonium ion impurities contained in the recovered disodium hydrogen phosphate was significantly reduced when compared with other processes.

### Example 4. Recovery of Phosphoric Acid By Adding Methanol

### Example 4-1. Recovery of Phosphoric Acid By Adding 100 ml of Methanol

A waste liquid based on the process of *O*-phosphoserine fermentation according to the present example had an ion composition (g/L) of 23.8 of sodium ions, 6.4 of ammonium ions, 15.3 of chlorine ions, 6.4 of sulfate ions, and 37.1 of phosphate ions. 1600 ml of water was evaporated from 2000 ml of an initial waste liquid. Then, 50% (w/w) aqueous solution of sodium hydroxide (48.0 ml), was added thereto until the pH reached 9.07 and 100 ml of methanol was added thereto. This solution had a phosphate ion concentration of 135.2 g/L. The solution was cooled down to 15°C to obtain disodium hydrogen phosphate heptahydrate. The slurry was filtered using a centrifugal basket filter and washed with pure water.

A weight of a final product was 182.7 g. The final product had an ion composition (g/kg) of 135.5 of sodium ions, 24.9 of ammonium ions, 13.1 of chlorine ions, 2.2 of sulfate ions, and 328.5 of phosphate ions. A moisture content of the final product was 49.6 wt%. A total recovery rate of phosphoric acid was 81.0 wt%.

### Example 4-2. Recovery of Phosphoric Acid By Adding 200 ml of Methanol

A waste liquid based on the process of *O*-phosphoserine fermentation according to the present example had an ion composition (g/L) of 23.9 of sodium ions, 6.4 of ammonium ions, 15.3 of chlorine ions, 3.9 of sulfate ions, and 37.3 of phosphate ions. 1600 ml of water was evaporated from 2000 ml of an initial waste liquid. Then, 50% (w/w) aqueous solution of sodium hydroxide (47.0 ml), was added thereto until the pH reached 9.04 and 200 ml of methanol was added thereto. This solution had a phosphate ion concentration of 115.2 g/L. The solution was cooled down to 15°C to obtain disodium hydrogen phosphate heptahydrate. The slurry was filtered using a centrifugal basket filter and washed with pure water.

A weight of a final product was 182.0 g. The final product had an ion composition (g/kg) of 122.9 of sodium ions, 38.9 of ammonium ions, 15.9 of chlorine ions, 2.4 of sulfate ions, and 328.2 of phosphate ions. A moisture content of the final product was 46.6 wt%. A total recovery rate of phosphoric acid was 80.2 wt%.

Therefore, it was confirmed that the recovery rate of phosphoric acid increased by adding methanol after adjusting the pH or in the pH-adjusting step.

### Example 5. Recovery of Phosphoric Acid by Two-stage Crystallization

Based on International Patent Application Publication No. WO 2014/182125, O-phosphoserine fermentation and enzymatic conversion reaction of L-cysteine were performed. After these processes, the waste liquid of the process had an ion composition (g/L) of 21.7 of sodium ions, 4.4 of ammonium ions, 16.0 of chlorine ions, 30.1 of sulfate ions, and 30.3 of phosphate ions. 16.7 L of water was evaporated from 20 L of an initial waste liquid, 50% (w/w) aqueous solution of sodium hydroxide (0.4 L), was added thereto until the pH reached 9.00. This solution had a phosphate ion concentration of 162.1 g/L. The solution was cooled down to 15°C to obtain disodium hydrogen phosphate heptahydrate. The slurry of disodium hydrogen phosphate heptahydrate was filtered using a centrifugal basket filter and washed with pure water. A weight of the recovered disodium hydrogen phosphate heptahydrate was 1339.0 g. The product had an ion composition (g/kg) of 133.5 of sodium ions, 18.7 of ammonium ions, 5.3 of chlorine ions, 1.3 of sulfate ions, and 323.4 of phosphate ions. A moisture content of the final product was 48.2 wt%.

After filtering the slurry, a filtrate had an ion composition (g/L) of 13.3 of sodium ions, 6.8 of ammonium ions, 98.9 of chlorine ions, 16.8 of sulfate ions, and 40.2 of phosphate ions. 1.1 g of disodium hydrogen phosphate dodecahydrate (sample prepared in Example 1-3) was added to 3.2 L of the remaining filtrate and stirred at 15°C for 4 hours. The slurry of disodium hydrogen phosphate dodecahydrate was filtered using a centrifugal basket filter and washed with pure water.

A weight of the recovered disodium hydrogen phosphate dodecahydrate was 299.0 g. The product had an ion composition (g/kg) of 128.2 of sodium ions, 6.1 of chlorine ions, 3.8 of sulfate ions, and 239.5 of phosphate ions. A moisture content was 61.3 wt%. A final recovery rate of phosphoric acid of the above-described two-step process was 83.2 wt%. When the recovered disodium hydrogen phosphate heptahydrate was mixed with the disodium hydrogen phosphate dodecahydrate and reused as a precursor of fermentation of *O*-phosphoserine, the fermentation process proceeded without any problem.

### Example 6. Preparation of O-phosphoserine Using Recovered Disodium Hydrogen Phosphate

A strain deposited under accession No. KCCM11103P and disclosed in International Patent Application Publication No. WO 2012/053794 was incubated on an MMYE agar medium plate containing 50 µg/ml spectinomycin (2 g/L glucose, 2 mM magnesium sulfate, 0.1 mM calcium chloride, 6 g/L sodium pyrophosphate, 0.5 g/L sodium chloride, 3 g/L disodium hydrogen phosphate recovered according to the present disclosure, 10 g/L yeast extract, and 18 g/L agar) at 33°C for 24 hours. The resulting cells were scrapped from 1/10 of the area of each agar plate, inoculated into 50 ml of a flask seed medium containing 50 µg/ml spectinomycin (10 g/L glucose, 0.5 g/L magnesium sulfate, 3 g/L potassium dihydrogen phosphate, 10 g/L yeast extract, 0.5 g/L sodium chloride, 1.5 g/L ammonium chloride, 12.8 g/L sodium pyrophosphate, and 1 g/L glycine) in a baffle flask and incubated at 30°C for 6 hours at 200 rpm.

After the seed culture was completed, the seed culture medium in an amount of 16% of the volume of a main culture medium was inoculated onto a 1 L small fermentor filled with 300 ml of the main culture medium, followed by incubation at 33°C and pH 7.0. During incubation, the pH was adjusted to 7.0 by adding ammonia water. Upon depletion of glucose from the medium, fed-batch culture was performed by adding a 520 g/L glucose solution and a 300 g/L disodium hydrogen phosphate recovered according to the present disclosure thereto. After performing incubation for 80 hours, O-phosphoserine at a concentration of 29.3 g/L was measured by HPLC.

### Comparative Example. Preparation of O-phosphoserine Using Pure Phosphoric Acid

The stain deposited under accession No. KCCM11103P was incubated in the seed medium and the main medium in the same manner as in Example 6. Upon depletion of glucose, fed-batch culture was performed by adding 520 g/L glucose solution and 200 g/L pure phosphoric acid thereto. After performing incubation for 80 hours, *O*-phosphoserine at a concentration of 29.5 g/L was measured by HPLC.

Therefore, it was confirmed that, according to the method of recovering phosphoric acid from a fermentation medium and reusing the same, a phosphate can be recovered by crystallizing the phosphate in a fermentation broth or a fermentation waste liquid, and by using the recovered phosphate in fermentation, a fermentation product can be obtained in an amount similar to that obtained using pure phosphoric acid, and thus the method according to the present disclosure can be used in a fermentation processes very effectively.

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

## Claims

1. A method of recovering phosphoric acid from a fermentation broth or a fermentation waste liquid and reusing the recovered phosphoric acid, the method comprising:
(a) concentrating a fermentation broth or a fermentation waste liquid containing phosphoric acid (concentrating step);
(b) adjusting the pH of the concentrate in the range of 8 to 11 (pH-adjusting step);
(c) crystallizing a phosphate from the concentrate having the adjusted pH and separating the crystalized phosphate from a mother liquor (crystallizing step); and
(d) using the crystallized phosphate in a fermentation medium as a phosphoric acid source (reusing step).

2. A method of recovering phosphoric acid from a fermentation broth or a fermentation waste liquid and reusing the recovered phosphoric acid, the method comprising:
(a) producing *O*-phosphoserine (OPS) using a microorganism in a fermentation medium containing phosphoric acid (OPS fermentation step);
(b) preparing a fermentation broth of cysteine or a derivative thereof by reacting *O*-phosphoserine produced in the step (a) with a sulfide in the presence of *O*-phosphoserine sulfhydrylase (OPSS) or a microorganism expressing the OPSS (converting step);
(c) concentrating the fermentation broth; or a fermentation waste liquid obtained by removing cysteine or a derivative thereof from the fermentation broth (concentrating step);
(d) adjusting the pH of the concentrate in the range of 8 to 11 (pH-adjusting step);
(e) crystallizing a phosphate from the concentrate having the adjusted pH and separating the crystalized phosphate from a mother liquor (crystallizing step); and
(f) using the crystallized phosphate in a fermentation medium as a phosphoric acid source (reusing step).

3. A method of recovering phosphoric acid from a fermentation broth or fermentation waste liquid and reusing the recovered phosphoric acid, the method comprising:
(a) producing *O*-phosphohomoserine (OPHS) using a microorganism in a fermentation medium containing phosphoric acid (OPHS fermentation step);
(b) preparing a fermentation broth of methionine or a derivative thereof by reacting *O*-phosphohomoserine produced in the step (a) with a sulfide in the presence of *O*-phosphohomoserine-dependent methionine synthase or a microorganism expressing the *O*-phosphohomoserine-dependent methionine synthase (converting step);
(c) concentrating the fermentation broth; or a fermentation waste liquid obtained by removing methionine or a derivative thereof from the fermentation broth (concentrating step);
(d) adjusting the pH of the concentrate in the range of 8 to 11 (pH-adjusting step);
(e) crystallizing a phosphate from the concentrate having the adjusted pH and separating the crystalized phosphate from a mother liquor (crystallizing step); and
(f) using the crystallized phosphate in a fermentation medium as a phosphoric acid source (reusing step).

4. A method of recovering phosphoric acid, the method comprising:
(a) concentrating a fermentation waste liquid containing phosphoric acid (concentrating step);
(b) adjusting the pH of the concentrate in the range of 8 to 11 (pH-adjusting step);
(c) crystallizing a phosphate from the concentrate having the adjusted pH (crystallizing step); and
(d) separating the crystallized phosphate from a mother liquor (separating step).

5. The method of claim **1,** wherein the fermentation broth is (i) an *O*-phosphoserine (OPS) fermentation broth, (ii) an *O*-phosphohomoserine (OPHS) fermentation broth, or (iii) a fermentation broth comprising an amino acid or a derivative thereof, prepared by adding a converting enzyme or a microorganism expressing the converting enzyme; and a sulfide to the fermentation broth, and
the fermentation waste liquid is a waste liquid obtained by removing the amino acid or a derivative thereof from the fermentation broth.

6. The method of claim **2 or 3,** wherein the sulfide is at least one selected from the group consisting of CH₃SH, Na₂S, NaSH, (NH₄)₂S, H₂S, and Na₂S₂O₃.

7. The method of claim **1 or** 4, wherein the fermentation waste liquid is obtained by removing the amino acid or a derivative thereof from the fermentation broth.

8. The method of any one of claims **1 to 4,** wherein the pH-adjusting step is performed by adding a hydroxide to the concentrate.

9. The method of claim 8, wherein the hydroxide is sodium hydroxide or an aqueous solution of sodium hydroxide.

10. The method of any one of claims **1 to 4,** further comprising cooling the concentrate having the adjusted pH between the pH-adjusting step and the crystallizing step or in the crystallizing step.

11. The method of any one of claims **1 to 4,** further comprising adding phosphate crystals, as crystal nuclei, between the pH-adjusting step and the crystallizing step or in crystallizing step.

12. The method of any one of claims **1 to 4,** wherein the phosphate comprises at least one selected from the group consisting of trisodium phosphate, disodium hydrogen phosphate, sodium dihydrogen phosphate, and a hydrate thereof.

13. The method of claim **12,** wherein the hydrate is disodium hydrogen phosphate heptahydrate or disodium hydrogen phosphate dodecahydrate.

14. The method of any one of claims **1 to 4,** further comprising crystallizing the phosphate from the mother liquor (second crystallizing step).

15. The method of claim **14,** wherein the second crystallizing step comprises (i) concentrating the mother liquor (re-concentrating step), (ii) adjusting the pH of the mother liquor in the range of 8 to 11 (pH re-adjusting step), and (iii) crystallizing the phosphate from the mother liquor having the adjusted pH and separating the crystalized phosphate therefrom (re-crystallizing step).

16. The method of claim **15,** further comprising adding phosphate crystals, as crystal nuclei, to the mother liquor between the pH re-adjusting step and the re-crystallizing step or in the re-crystallizing step.

17. The method of any one of claims **1 to 4,** further comprising adjusting the pH of the fermentation broth or fermentation waste liquid in the range of 8 to 11 before the concentrating step.

18. The method of any one of claims **1 to 4,** further comprising adding an alcohol in the pH-adjusting step; or between the pH-adjusting step and the crystallizing step.

19. The method of claim **18,** wherein the alcohol is methanol, ethanol, propanol, butanol, or any combination thereof.
